## Europäisches Patentamt
## European Patent Office
## Office européen des brevets

(11) Numéro de publication: **0 320 332**
**A1**

# DEMANDE DE BREVET EUROPEEN

(21) Numéro de dépôt: **88402971.1**

(22) Date de dépôt: **25.11.88**

(51) Int. Cl.⁴: **A 61 L 27/00**

(30) Priorité: **08.12.87 FR 8717033**

(43) Date de publication de la demande:
**14.06.89 Bulletin 89/24**

(84) Etats contractants désignés:
**BE CH DE ES GB IT LI NL**

(71) Demandeur: **Bouvet, Jean-Claude**
**11 Route de Corbeil Mondeville**
**F-91590 La Ferte Alais (FR)**

(72) Inventeur: **Bouvet, Jean-Claude**
**11 Route de Corbeil Mondeville**
**F-91590 La Ferte Alais (FR)**

(74) Mandataire: **Flavenot, Bernard**
**Société ABRITT 17, rue du Docteur Charcot La Norville**
**F-91290 Arpajon (FR)**

(54) **Rotule pour prothèse.**

(57) La présente invention concerne les rotules pour prothèses.

La rotule selon la présente invention se caractérise essentiellement par le fait qu'elle comporte deux parties mâle et femelle, la partie mâle comprenant une tête sphérique 13 et des moyens 9,10,11,... pour monter fixement la tête sur une patte de fixation 4, la partie femelle comprenant une cupulle 17 d'une forme sphérique concave complémentaire de la tête sphérique, la tête sphérique étant réalisée dans un matériau ayant une structure cristalline, comme un monocristal, tel que du rubis, saphir ou diamant.

Application à la réalisation, notamment, des prothèses de hanche ou analogues..

Bundesdruckerei Berlin

EP 0 320 332 A1

## Description

### Rotule pour prothèse

La présente invention concerne les rotules pour prothèses telles que celles qui sont implantées dans le corps humain quand une articulation est détériorée, par exemple suite à une maladie ou à un accident.

Les chirurgiens sont très souvent amenés à pratiquer des opérations qui consistent à implanter des prothèses. Parmi celles-ci, il en existe une qui est bien connue et très courante, c'est celle de la hanche. En effet, suite à des accidents ou des maladies, notamment les personnes âgées souffrent par exemple d'une détérioration de la tête du fémur qu'il est alors nécessaire de remplacer par des moyens comme une prothèse à rotule. Cette rotule comporte une partie mâle généralement constituée par une portion de sphère et une partie femelle constituée par une cupulle de même rayon que la sphère, la partie mâle étant maintenue dans cette cupulle par tous moyens, notamment par les tendons et les muscles du patient sur lequel est implantée la prothèse.

Les prothèses qui sont réalisées sur ce modèle comprennent très schématiquement deux parties, une tête sphérique montée sur une patte par tous moyens, la patte étant destinée à être implantée dans un os comme le fémur, pour le cas d'une prothèse de hanche, et une cupulle de forme complémentaire à la tête qui est, elle, destinée à être implantée sur le bassin.

Généralement, la réalisation de la patte de fixation ne pose pas de problème, notamment quant à son état de surface ou la nature du matériau la constituant. Par contre, la réalisation de la tête de rotule, en l'occurrence la portion de sphère, présente beaucoup plus de difficultés. Les premières têtes ont été réalisées dans des matériaux métalliques. Mais, devant certains problèmes rencontrés par les hommes de l'art, ceux-ci ont pensé utiliser des céramiques, c'est-à-dire des matériaux obtenus en noyant des poudres de matières solides dans des adjuvants organiques comme des résines adaptées et en portant ensuite le mélange à haute température.

Actuellement, c'est cette réalisation qui est le plus souvent utilisée, bien que les rotules ainsi obtenues n'aient pas une solidité qui donne toute satisfaction.

Il est en effet souvent constaté que, lorsqu'une telle sphère subit un choc important, par exemple lors d'une chute du patient sur lequel elle est implantée, elle éclate.

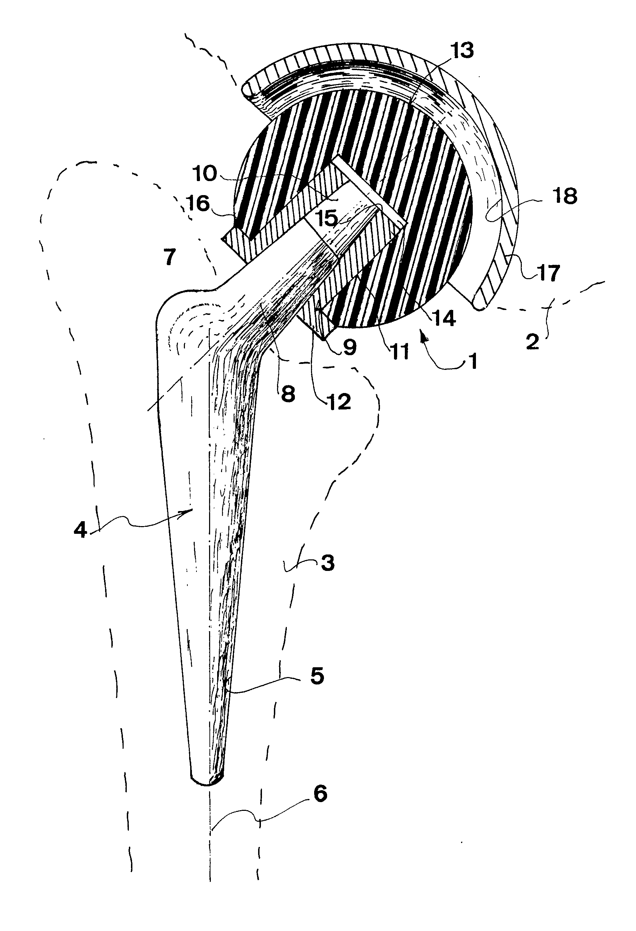

Pour essayer de pallier cet inconvénient, il a été réalisé des dispositifs de liaison spéciaux peur associer ces têtes de rotules aux pattes de fixation, par exemple comme celui qui est illustré sur la figure 1 de la Demande de Brevet Français publiée sous le numéro 2 398 490.

Bien que, par ailleurs, les moyens de la coopération en rotation de la tête de rotule avec la cupulle donnent des résultats satisfaisants, on constate pourtant, encore, des problèmes de solidité.

La présente invention a pour but de réaliser une rotule pour prothèse qui allie les qualités de friction des matériaux comme les céramiques, et de solidité du métal, et qui, de plus, ne présente pas de difficultés majeures de réalisation.

Plus précisément, la présente invention a pour objet une rotule de prothèse comportant deux parties mâle et femelle, la partie mâle comprenant une tête sphérique et des moyens pour monter fixement ladite tête sur une patte de fixation, la partie femelle comprenant une cupulle d'une forme sphérique concave complémentaire de la tête sphérique, caractérisée par le fait que ladite tête sphérique est réalisée dans un matériau à structure cristalline.

Selon une autre caractéristique de la présente invention, ledit matériau est un monocristal.

Selon une autre caractéristique de la présente invention, ledit monocristal est choisi parmi l'un des cristaux suivants : rubis, saphir, diamant.

D'autres caractéristiques et avantages de la présente invention apparaîtront au cours de la description suivante donnée en regard du dessin annexé à titre illustratif, mais nullement limitatif, dans lequel la figure unique représente, vu en coupe, un mode de réalisation d'une rotule selon l'invention, dans le cas d'une prothèse de hanche.

La figure unique représente donc, à titre d'exemple, une rotule 1 pour une prothèse comme celle de hanche 2 destinée à être fixée sur un fémur 3.

Cette prothèse de hanche comprend schématiquement une patte de fixation 4 apte à être fixée dans un orifice 5 pratiqué suivant l'axe 6 du fémur 3.

Les moyens de fixation de cette patte 4 dans le fémur 3 sont connus en eux-mêmes et ne seront pas plus amplement décrits ci-après.

A son extrémité émergente 7, la patte de fixation 4 comprend généralement une partie conique 8 sur laquelle est apte à être emboîté un manchon de liaison 9 comportant un orifice traversant 10 de forme conique complémentaire de la partie conique 8 de la tige 4.

La paroi extérieure 11 de ce manchon de liaison 9 est avantageusement de forme cylindrique et délimitée à une certaine distance de la section la plus faible de la partie conique 8, par exemple, par un épaulement 12 débordant latéralement.

Sur ce manchon est alors enfichée une portion de sphère 13 comportant un logement en creux 14 d'une section intérieure au moins égale à celle de la paroi extérieure 11 du manchon de liaison et d'une profondeur au moins égale à la distance séparant le sommet 15 de ce manchon 9 de l'épaulement 12.

De cette façon, la portion de sphère 13 est enfichée sur le manchon 9 jusqu'à ce que les bords 16 du logement 14 viennent au contact de l'épaulement 12. Elle est alors solidarisée au manchon par tous moyens, par exemple par colle, brasure, etc, selon la nature du matériau dans lequel elle est réalisée.

La rotule 1 comporte une seconde partie constituée par une cupulle 17 qui est en fait une partie de calotte sphérique concave 18 d'une sphéricité complémentaire de celle de la portion de sphère 13, de telle façon que la portion de sphère puisse épouser cette surface concave 18 dans un emboîtement mâle-femelle parfait.

La technique générale de la rotule décrite ci-dessus pour la réalisation, par exemple, d'une prothèse de hanche, et telle qu'illustrée sur la figure 1, est bien connue en elle-même. Cependant, dans le but d'améliorer la qualité du frottement entre les deux parties de la rotule et d'augmenter la durée de vie d'une telle rotule, et donc de la prothèse la comportant, selon la caractéristique principale de l'invention, la portion de sphère 13 est réalisée dans un matériau ayant une structure cristalline, par exemple un monocristal d'alumine comportant différentes quantités de matériaux de doppage comme du fer, du chrome. Pour des applications de haute qualité, il peut même être utilisé un cristal de diamant. La partie femelle de la rotule constituée par la cupulle 17 peut être réalisée en matière plastique apte à être implantée dans le corps humain, par exemple l'une des matières couramment utilisées dans ce domaine.

En fait, les cristaux utilisés pour la réalisation des têtes sphériques 13 sont relativement faciles à obtenir, notamment avec un matériau à base d'alumine, le cristal total nécessaire pour la réalisation de la portion de sphère pouvant être obtenu par croissance à partir d'un germe du monocristal, par exemple par la méthode Czochralski.

Les expérimentations faites par l'inventeur ont montré que de telles réalisations ont donné toute satisfaction, notamment quant aux qualités de finition et de solidité, et surtout, sont beaucoup plus fiables que celles de l'art antérieur.

## Revendications

1. Rotule (1) de prothèse comportant deux parties mâle et femelle, la partie mâle comprenant une tête sphérique (13) et des moyens (9,10,11,...) pour monter fixement ladite tête sur une patte de fixation (4), la partie femelle comprenant une cupulle (17) d'une forme sphérique concave (18) complémentaire de la tête sphérique, caractérisée par le fait que ladite tête sphérique (13) est réalisée dans un matériau ayant une structure cristalline.

2. Rotule selon la revendication 1, caractérisée par le fait que ledit matériau ayant une structure cristalline est un monocristal.

3. Rotule selon la revendication 2, caractérisée par le fait que ledit matériau de structure cristalline est un monocristal à base d'alumine cristallisée.

4. Rotule selon la revendication 2, caractérisée par le fait que ledit monocristal est obtenu par croissance à partir d'un germe dudit monocristal.

5. Rotule selon la revendication 2, caractérisée par le fait que ledit monocristal est choisi parmi l'un des cristaux suivants, rubis, saphir, diamant.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | DE-A-2 641 695  (KYOTO CERAMIC K.K.) * Revendications 1-3; page 8, lignes 11-23 * ----- | 1-5 | A 61 L  27/00 |

**DOMAINES TECHNIQUES RECHERCHES (Int. Cl.4)**

A 61 L

Le présent rapport a été établi pour toutes les revendications

| Lieu de la recherche | Date d'achèvement de la recherche | Examinateur |
|---|---|---|
| LA HAYE | 14-03-1989 | NEILL M.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
...............................................................................................
& : membre de la même famille, document correspondant

EPO FORM 1503 03.82 (P0402)